(19) Europäisches Patentamt
European Patent Office
Office européen des brevets

(11) **EP 2 412 289 A1**

(12) **EUROPEAN PATENT APPLICATION**
published in accordance with Art. 153(4) EPC

(43) Date of publication:
**01.02.2012 Bulletin 2012/05**

(51) Int Cl.:
***A61B 1/00*** *(2006.01)*

(21) Application number: **09842317.1**

(22) Date of filing: **09.10.2009**

(86) International application number:
**PCT/JP2009/067666**

(87) International publication number:
**WO 2010/109707 (30.09.2010 Gazette 2010/39)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.03.2009 JP 2009072167**

(71) Applicant: **OLYMPUS MEDICAL SYSTEMS CORP. Tokyo 151-0072 (JP)**

(72) Inventor: **TAKEI, Shunji Tokyo 151-0072 (JP)**

(74) Representative: **Schicker, Silvia Wuesthoff & Wuesthoff Patent- und Rechtsanwälte Schweigerstrasse 2 81541 München (DE)**

(54) **FLUORESCENCE OBSERVATION DEVICE**

(57) A fluorescence observation apparatus according to the present invention includes a light source section that can emit a plurality of excitation light beams of different wavelength bands and select excitation light beams to be emitted to an object where there are a first fluorescent substance and a second fluorescent substance from among the plurality of excitation light beams, a variable filter section that selectively allows at least part of the wavelength band of returning light from the object to pass, a photo-detecting section that detects the light that has passed through the variable filter section, a calculation processing section that carries out calculations to determine a wavelength band of the excitation light emitted from the light source section and a transmission wavelength band in the variable filter section based on intensity characteristics of the plurality of excitation light beams, fluorescence characteristics of the first fluorescent substance and fluorescence characteristics of the second fluorescent substance and a control section that performs control of causing the light source section to interlock with the variable filter section based on the calculation result of the calculation processing section.

**FIG.5**

**Description**

Technical Field

The present invention relates to a fluorescence observation apparatus, and more particularly, to a fluorescence observation apparatus for observing fluorescent light beams emitted from a plurality of fluorescence probes.

Background Art

Cancer diagnosis techniques using molecular targeting agents are becoming a focus of attention in recent years. To be more specific, for example, a technique of scattering or injecting a fluorescence probe (fluorescence agent) targeting at biological protein that is developed specifically in cancer cells and then identifying the presence/absence of cancer based on fluorescent light emitted from the target region is under study in recent years. Such a technique is useful for early detection of cancer in the field of the digestive tract.

Furthermore, as an application of the aforementioned technique, a technique is being proposed which is designed to scatter or inject a plurality of types of fluorescence probes into a target region of a living body and observe the developed state of a plurality of types of biological protein corresponding to the plurality of types of fluorescence probes in a composite manner based on fluorescent light of a plurality of wavelengths emitted from the target region. Such a technique is considered useful for estimation of stages of cancer, prediction of risk of cancer invasion and prediction of risk of cancer metastasis or the like.

For example, Japanese Patent Application Laid-Open Publication No.2008-43396 discloses an endoscope system that makes observations by scattering or injecting a plurality of types of fluorescence probes into a target region of a living body, configured to be able to acquire a fluorescence image (image of a fluorescent light distribution) for each fluorescence probe by carrying out calculation processing based on a relationship between the intensity of fluorescent light and concentration of the fluorescence probe obtained during the observations.

Incidentally, when performing an observation by scattering or injecting a plurality of types of fluorescence probes to a target region of a living body, so-called cross-talk phenomenon occurs which is a phenomenon of overlap of fluorescent light bands of a plurality of wavelengths emitted from the target region. Such cross-talk phenomenon is regarded as a problem as relating to, e.g., reduced contrast of an observation image.

A technique recited in Japanese Patent Application Laid-Open Publication No. 2008-43396, on the other hand, is capable of decreasing the cross-talk phenomenon through combination of predetermined fluorescence probes, while in contrast has a problem of difficulty in obtaining the effect for decreasing the cross-talk phenomenon when the combination of predetermined fluorescence probes is not adopted. The technique recited in Japanese Patent Application Laid-Open Publication No. 2008-43396 has another problem that the effect of decreasing the cross-talk phenomenon is hard to be obtained when a new fluorescence probe is used whose detailed fluorescence characteristics are unknown.

The present invention was made in view of the above-described circumstance and has an objective to provide a fluorescence observation apparatus capable of decreasing, through combination of various fluorescence probes, the cross-talk phenomenon that occurs during observation of fluorescent lights respectively emitted corresponding to a plurality of fluorescence probes.

Disclosure of Invention

Means for Solving the Problem

A fluorescence observation apparatus according to the present invention includes a light source section that can emit a plurality of excitation light beams of different wavelength bands and select excitation light beams to be emitted to an object where there are a first fluorescent substance and a second fluorescent substance from among the plurality of excitation light beams, a variable filter section that selectively allows at least part of the wavelength band of returning light from the object to pass, a photo-detecting section that detects the light that has passed through the variable filter section, a calculation processing section that carries out calculations to determine a wavelength band of the excitation light emitted from the light source section and a transmission wavelength band in the variable filter section based on intensity characteristics of the plurality of excitation light beams, fluorescence characteristics of the first fluorescent substance and fluorescence characteristics of the second fluorescent substance and a control section that performs control of causing the light source section to interlock with the variable filter section based on the calculation result of the calculation processing section.

A fluorescence observation apparatus according to the present invention includes a light source section that can emit a plurality of excitation light beams of different wavelength bands and select excitation light beams to be emitted to an object where there are a first fluorescent substance and a second fluorescent substance from among the plurality of excitation light beams, a variable filter section that selectively allows at least part of the wavelength band of returning light from the object to pass, a photo-detecting section that detects the light that has passed through the variable filter section, an analysis section that acquires a fluorescence image corresponding to the fluorescent light emitted from the first fluorescent substance, a fluorescence image corresponding to the fluorescent light emitted from the second fluorescent substance and a fluorescence image corresponding to the fluorescent light emitted from a mixed region of the first fluorescent substance and the second fluorescent substance based on the detection result in the photo-detecting section and determines a wavelength band of the excitation light emitted from the light source section and a transmission wavelength band in the variable filter section based on luminance of each of the fluorescence images and a control section that performs control of causing the light source section to interlock with the variable filter section based on the analysis result of the analysis section.

Brief Description of the Drawings

Fig. 1 is a diagram illustrating a configuration of main parts of an endoscope system according to a first embodiment of the present invention;
Fig. 2 is a diagram illustrating an example of another light source apparatus applicable to the endoscope system in Fig. 1;
Fig. 3 is a diagram illustrating a configuration of main parts of an endoscope system according to a second embodiment of the present invention;
Fig. 4 is a diagram illustrating an example of object to which two types of fluorescence agents are applied;
Fig. 5 is a diagram illustrating a case where the intensity of the fluorescent light emitted from the first fluorescence agent in the object illustrated in Fig. 4 reaches a maximum value;
Fig. 6 is a diagram illustrating a case where the intensity of the fluorescent light emitted from the second fluorescence agent in the object illustrated in Fig. 4 reaches a maximum value;
Fig. 7 is a schematic view illustrating an example of the fluorescence image obtained in the case shown in Fig. 5;
Fig. 8 is a schematic view illustrating an example of the fluorescence image obtained in the case shown in Fig. 6;
Fig. 9 is a diagram illustrating an example of luminance distribution having only one peak corresponding to the fluorescent light emitted from the first fluorescence agent;
Fig. 10 is a diagram illustrating an example of luminance distribution having only one peak corresponding to the fluorescent light emitted from the second fluorescence agent; and
Fig. 11 is a diagram illustrating an example of another light source apparatus applicable to the endoscope system in Fig. 3.

Best Mode for Carrying Out the Invention

Hereinafter, embodiments of the present invention will be described with reference to the accompanying drawings.

(First embodiment)

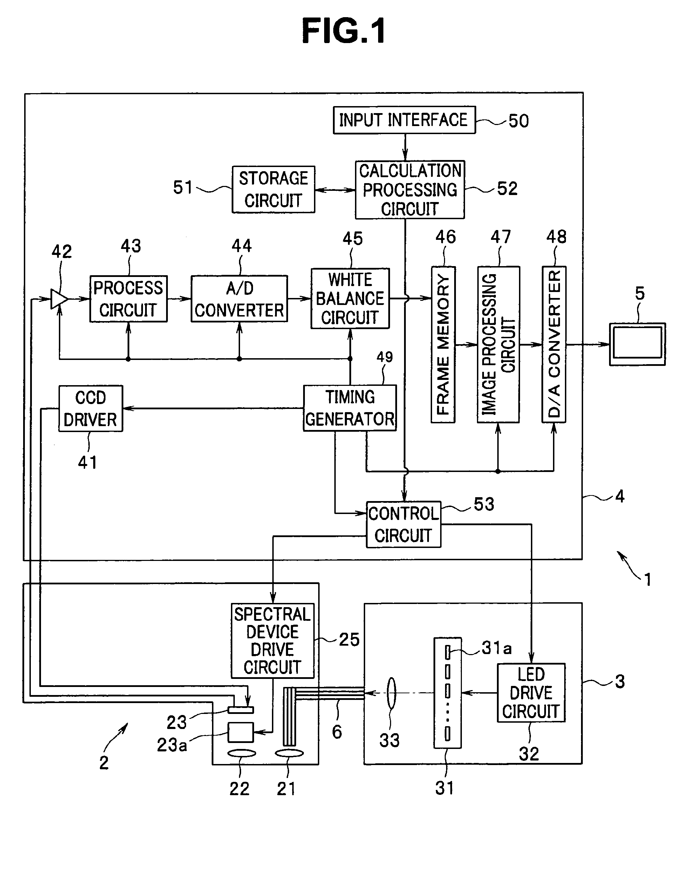

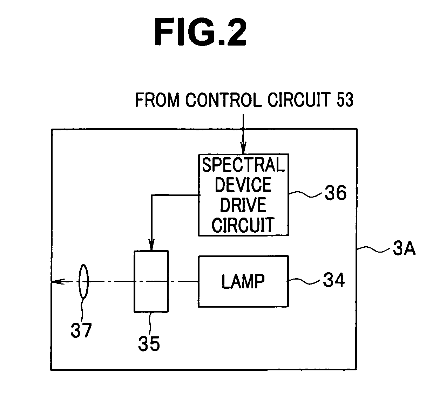

Fig. 1 and Fig. 2 are related to a first embodiment of the present invention.
As shown in Fig. 1, an endoscope system 1 is configured by including an endoscope 2 that can be inserted into a living body to acquire an object image of living tissue or the like existing in the living body and output the acquired object image as an image pickup signal, a light source apparatus 3 that supplies light to be emitted to the object to the endoscope 2 via a light guide 6 inserted in the endoscope 2, a processor 4 that performs signal processing according to the image pickup signal outputted from the endoscope 2 and outputs the image pickup signal subjected to the signal processing as a video signal and a monitor 5 that displays the object image acquired by the endoscope 2 based on the video signal outputted from the processor 4.
The endoscope 2 is configured by including an illumination optical system 21 that outputs the light supplied from the light source apparatus 3 and transmitted by the light guide 6 to the object, an objective optical system 22 that forms an object image, a CCD (charge coupled device) 23 whose image pickup surface is disposed at the image forming position of the objective optical system 22, a spectral device 23a disposed before the CCD 23 and a spectral device drive circuit 25 that drives the spectral device 23a based on the control of the processor 4.
The spectral device 23a provided with the function as a variable filter section is configured to change the own transmission wavelength band according to a drive signal from the spectral device drive circuit 25 and thereby selectively allow only light of a wavelength band corresponding to the drive signal to pass out of returning light inputted through the objective optical system 22.

The light source apparatus 3 includes an LED light source section 31, an LED drive circuit 32 and a condensing optical system 33 that condenses light emitted from the LED light source section 31 and supplies the condensed light to the light guide 6.

The LED light source section 31 is configured of a plurality of LEDs 31a which emit light of different wavelength bands (and center wavelengths) arranged in an array form.

The LED drive circuit 32 selects and drives an LED 31a that corresponds to the control of the processor 4 out of the plurality of LEDs 31a in the LED light source section 31.

As shown in Fig. 1, the processor 4 includes a CCD driver 41 that drives the CCD 23, an amplifier 42 that amplifies an image pickup signal outputted from the CCD 23, a process circuit 43 that applies signal processing such as correlation double sampling to the image pickup signal outputted from the amplifier 42 and an A/D converter 44 that converts the image pickup signal outputted from the process circuit 43 to a digital image signal.

Furthermore, as shown in Fig. 1, the processor 4 also includes a white balance circuit 45, a frame memory 46, an image processing circuit 47, a D/A converter 48 and a timing generator 49.

The white balance circuit 45 applies white balance processing to the image signal from the A/D converter 44 based on input timing of a timing signal from the timing generator 49 and outputs the image signal subjected to the white balance processing to the frame memory 46.

The frame memory 46 sequentially stores image signals outputted from the white balance circuit 45 in units of one frame.

The image processing circuit 47 reads an image signal stored in the frame memory 46 based on input timing of a timing signal from the timing generator 49. The image processing circuit 47 applies predetermined image processing to the read image signal and outputs the image signal to the D/A converter 48.

The D/A converter 48 converts the image signal outputted from the image processing circuit 47 to an analog video signal based on input timing of a timing signal from the timing generator 49 and outputs the video signal.

The timing generator 49 generates timing signals indicating operation timing of the respective sections and then outputs the timing signals to the CCD driver 41, the amplifier 42, the process circuit 43, the A/D converter 44, the white balance circuit 45, the image processing circuit 47, the D/A converter 48 and a control circuit 53 respectively.

Furthermore, as shown in Fig. 1, the processor 4 also includes an input interface 50 provided with an input device such as a keyboard, a storage circuit 51, a calculation processing circuit 52 and the control circuit 53.

The storage circuit 51 provided with the function as a storage section stores predetermined parameters associated with each fluorescence agent (fluorescence probe) and a function indicating a correlation between a wavelength and intensity of light (excitation light) emitted from the LED light source section 31.

The calculation processing circuit 52 reads the function indicating the correlation between the wavelength and intensity of light (excitation light) emitted from the LED light source section 31 and the parameters corresponding to the information on a fluorescence agent inputted through the input interface 50 from the storage circuit 51. Furthermore, the calculation processing circuit 52 performs calculation processing based on each value read from the storage circuit 51 and thereby acquires a center wavelength of excitation light and a detection wavelength band of fluorescent light in combination of fluorescence agents inputted through the input interface 50. The calculation processing circuit 52 outputs the calculation results of the center wavelength of excitation light and the detection wavelength band of fluorescent light to the control circuit 53.

The control circuit 53 performs control over the LED drive circuit 32 to sequentially switch between the LEDs 31a to emit light based on input timing of a timing signal from the timing generator 49 and the center wavelength of excitation light calculated by the calculation processing circuit 52. Furthermore, the control circuit 53 performs control over the spectral device drive circuit 25 to sequentially switch between transmission wavelength bands of the spectral device 23a based on input timing of a timing signal from the timing generator 49 and the detection wavelength band of fluorescent light calculated by the calculation processing circuit 52.

Furthermore, the control circuit 53 synchronizes the timing of control over the LED drive circuit 32 with the timing of control over the spectral device drive circuit 25 based on input timing of a timing signal from the timing generator 49. According to the control of the control circuit 53, the operation of the LED light source section 31 in the light source apparatus 3 is interlocked with the operation of the spectral device 23a in the endoscope 2.

Next, the operation of the endoscope system 1 will be described.

First, before using the endoscope system 1, the user inputs information (product name or substance name or the like) on n types (n≥2) of fluorescence agents used for fluorescence observation by operating the input interface 50 provided with the function as an input section.

On the other hand, upon detecting that the information on n types of fluorescence agents has been inputted to the input interface 50, the calculation processing circuit 52 reads a function I ($\lambda_{Ex}$) indicating a correlation between a wavelength $\lambda_{Ex}$ and intensity of excitation light emitted from the LED light source section 31 from the storage circuit 51.

Furthermore, upon detecting that the information on n types of fluorescence agents has been inputted to the input interface 50, the calculation processing circuit 52 reads parameters corresponding to the information. To be more specific, the calculation processing circuit 52 reads excitation efficiency $\gamma(\lambda_{Ex})$ corresponding to the wavelength $\lambda_{Ex}$ of the excitation light, a function $S(\lambda)$ indicating a correlation between a wavelength and intensity of fluorescent light emitted from a fluorescence agent, a detection limit wavelength $\lambda_{MAX}$ on the long wavelength side of the fluorescent light emitted from the fluorescence agent and a margin value $\Delta$ for preventing the wavelength band of the excitation light from overlapping with the detection wavelength band of the fluorescent light as parameters corresponding to the information on each fluorescence agent inputted to the input interface 50.

Here, with regard to the wavelength $\lambda_{Ex}$ of the excitation light, a relationship of following equation (1) holds between the value of intensity $F(\lambda_{Ex})$ of the fluorescent light emitted from one fluorescence agent and each value read by the calculation processing circuit 52.

$$F\left(\lambda_{Ex}\right) = I\left(\lambda_{Ex}\right) \cdot \gamma\left(\lambda_{Ex}\right) \cdot \sum_{\lambda=\lambda_{Ex}+\Delta}^{\lambda_{MAX}} S(\lambda) \quad \cdot \cdot \cdot \quad (1)$$

The calculation processing circuit 52 substitutes each value read from the storage circuit 51 into the right side of equation (1) above, then sequentially changes the wavelength $\lambda_{Ex}$ of the excitation light, and thereby acquires the wavelength $\lambda_{Ex}$ the excitation light at which the intensity $F(\lambda_{Ex})$ of the fluorescent light reaches a maximum value for each fluorescence agent. Center wavelengths of the excitation light $\lambda_{Ex1}, \lambda_{Ex2}, ..., \lambda_{Exn}$ corresponding to the n types of fluorescence agents are acquired respectively through such calculation processing.

That is, the calculation processing circuit 52 acquires the center wavelength of the excitation light capable of optimally exciting each fluorescence agent used for fluorescence observations by performing calculation processing using equation (1) above.

Furthermore, for a fluorescence agent to which an ID number i (i=1,2, ..., n-1, n) is assigned, assuming the intensity of the excitation light at a wavelength $\lambda_{Exi}$ is $I_i(\lambda_{Exi})$, excitation efficiency at the wavelength $\lambda_{Exi}$ is $\gamma_i(\lambda_{Exi})$ and the detection wavelength when fluorescent light emitted from a fluorescence agent with the ID number i is $\lambda_{Em}(>\lambda_{Exi}+\Delta)$, the value of detected intensity of the fluorescence $F_i(\lambda_{Ex})$ is calculated by following equation (2).

$$F_i\left(\lambda_{Em}\right) = I_i\left(\lambda_{Exi}\right) \cdot \gamma_i\left(\lambda_{Exi}\right) \cdot S\left(\lambda_{Em}\right) \quad \cdot \cdot \cdot \quad (2)$$

Furthermore, a contribution rate $C_i$ corresponding to the proportion of detected intensity $F_i(\lambda_{Exm})$ of a fluorescent light beam emitted from the fluorescence agent with the ID number i out of the detected intensity of all fluorescent light beams emitted from n types of fluorescence agents $(F_1(\lambda_{Ex})+F_2(\lambda_{Ex})+...+F_n(\lambda_{Em}))$ is calculated by following equation (3).

$$C_i = F_i\left(\lambda_{Em}\right) / \left(F_1\left(\lambda_{Em}\right) + F_2\left(\lambda_{Em}\right) + ... + F_n\left(\lambda_{Em}\right)\right) \quad \cdot \cdot \cdot \quad (3)$$

The calculation processing circuit 52 acquires all detection wavelengths $\lambda_{Em}$ at which the contribution rate $C_i$ of the fluorescence agent with the ID number i exceeds a predetermined value $P_{th}$ using the wavelength $\lambda_{Exi}$ of the excitation light acquired through the aforementioned calculation processing and equations (2) and (3) above. In this way, the calculation processing circuit 52 acquires a detection wavelength band $\lambda_{bi}$ made up of a plurality of detection wavelengths $\lambda_{Em}$ used to detect fluorescent light emitted from a fluorescence agent with the ID number i.

That is, the calculation processing circuit 52 performs calculation processing using equations (2) and (3) above, and thereby acquires detection wavelength bands capable of optimally detecting fluorescent light beams emitted from the respective fluorescence agents used for fluorescence observations.

The calculation processing circuit 52 acquires the center wavelength $\lambda_{Exi}$ of the excitation light for exciting the fluorescence agent with the ID number i and the detection wavelength band $\lambda_{bi}$ of the fluorescent light emitted from the fluorescence agent with the ID number i as the calculation results of the aforementioned calculation processing. The calculation processing circuit 52 then associates center wavelengths $\lambda_{Ex1}, \lambda_{Ex2} ..., \lambda_{Exn}$ of excitation light beams with

detection wavelength bands $\lambda_{b1}, \lambda_{b2}, ..., \lambda_{bn}$ of fluorescent light beams in a one-to-one correspondence and outputs the association result to the control circuit 53.

Based on input timings of timing signals from the timing generator 49 and the center wavelengths $\lambda_{Ex1}, \lambda_{Ex}, ... , \lambda_{Exn}$ of the excitation light beams calculated by the calculation processing circuit 52, the control circuit 53 performs control over the LED drive circuit 32 so as to switch between (select) the LEDs 31a to emit light in that order. Furthermore, based on input timings of timing signals from the timing generator 49 and the detection wavelength bands $\lambda_{b1}, \lambda_{b2}, ..., \lambda_{bn}$ of the fluorescent light beams calculated by the calculation processing circuit 52, the control circuit 53 performs control over the spectral device drive circuit 25 so as to switch between (select) the transmission wavelength bands of the spectral device 23a in that order.

Furthermore, the control circuit 53 synchronizes the timing of control over the LED drive circuit 32 with the timing of control over the spectral device drive circuit 25 based on the input timing of the timing signal from the timing generator 49. Through such control by the control circuit 53, with regard to the fluorescence agent to which an ID number i is assigned, the operation of the LED light source section 31 of the light source apparatus 3 of emitting excitation light having the center wavelength $\lambda_{Exi}$ is interlocked with the operation of the spectral device 23a of the endoscope 2 of allowing light (fluorescence image) having the detection wavelength band $\lambda_{bi}$ to pass.

On the other hand, the user inserts an insertion portion of the endoscope 2 into the body cavity of the examinee and places the distal end portion of the endoscope 2 so that excitation light is emitted to a position where a desired object such as cancer exists.

Furthermore, the user scatters or injects n types of fluorescence agents whose information is inputted through the operation of the input interface 50 into the desired object using a treatment instrument or the like (not shown) having a shape and size that allows it to be inserted into the endoscope 2.

Every time the excitation light beam having the center wavelength $\lambda_{Ex1}, \lambda_{Ex2}, ..., \lambda_{Exn}$ is emitted to the desired object, n types of fluorescent light beams corresponding to the respective excitation light beams are sequentially emitted from the desired object.

The fluorescent light emitted from the desired object passes through the objective optical system 22 and the spectral device 23a and sequentially forms images on the image pickup surface of the CCD 23 as fluorescence images of the object having the detection wavelength bands $\lambda_{b1}, \lambda_{b2}, ..., \lambda_{bn}$.

The CCD 23 then sequentially generates image pickup signals corresponding to the fluorescence images of the object having the detection wavelength bands $\lambda_{b1}, \lambda_{b2}, ..., \lambda_{bn}$ and outputs the image pickup signals to the processor 4.

Each image pickup signal outputted from the CCD 23 is amplified by the amplifier 42, subjected to signal processing by the process circuit 43 and converted to a digital image signal by the A/D converter 44. Each image signal outputted from the A/D converter 44 is subjected to white balance processing by the white balance circuit 45 and then inputted to the frame memory 46.

The frame memory 46 stores image signals corresponding to the fluorescence images of the object having the detection wavelength bands $\lambda_{b1}, \lambda_{b2}, ..., \lambda_{bn}$ sequentially in units of one frame.

The image processing circuit 47 simultaneously reads latest image signals corresponding to one frame for the fluorescence images of the object having the detection wavelength bands $\lambda_{b1}, \lambda_{b2}, ..., \lambda_{bn}$ respectively based on input timings of timing signals from the timing generator 49 from the frame memory 46. The image processing circuit 47 then applies image processing such as coloring with different colors to each image signal read from the frame memory 46 and outputs each image signal to the D/A converter 48.

The D/A converter 48 converts each image signal outputted from the image processing circuit 47 to an analog video signal based on input timing of a timing signal from the timing generator 49 and outputs the video signal. Thus, the monitor 5 displays an image in which it is possible to visually recognize a distribution of fluorescent light beams emitted from n types of fluorescence agents used for fluorescence observations.

As described above, when making fluorescence observations using a plurality of fluorescence agents, the endoscope system 1 optimizes the center wavelength of the excitation light and the detection wavelength band of fluorescent light for each fluorescence agent, and can thereby acquire an image in which degradation of contrast caused by a cross-talk phenomenon is drastically suppressed. That is, the endoscope system 1 can reduce a cross-talk phenomenon generated when observing each corresponding fluorescent light emitted according to a plurality of fluorescence agents in various combinations of fluorescence probes.

The endoscope system 1 of the present embodiment may also be configured using a light source apparatus 3A shown in Fig. 2 instead of the light source apparatus 3 shown in Fig. 1.

The light source apparatus 3A includes a lamp 34 made up of a xenon lamp or the like, a spectral device 35 disposed on the optical path of the lamp 34, a spectral device drive circuit 36 that drives the spectral device 35 based on the control of the control circuit 53 and a condensing optical system 37 that condenses light that has passed through the spectral device 35 and supplies the light to the light guide 6.

The spectral device 35 has a configuration that allows only light having a wavelength band corresponding to the drive signal to pass by changing the own transmission wavelength band according to a drive signal from the spectral device drive circuit 36.

That is, according to the configuration using the light source apparatus 3A shown in Fig. 2, by performing control over the spectral device drive circuit 36 so as to generate excitation light having center wavelengths $\lambda_{Ex1}$, $\lambda_{Ex2}$, ..., $\lambda_{Exn}$ in that order, it is possible to obtain effects substantially the same as those of the configuration using the light source apparatus 3 shown in Fig. 1.


(Second embodiment)

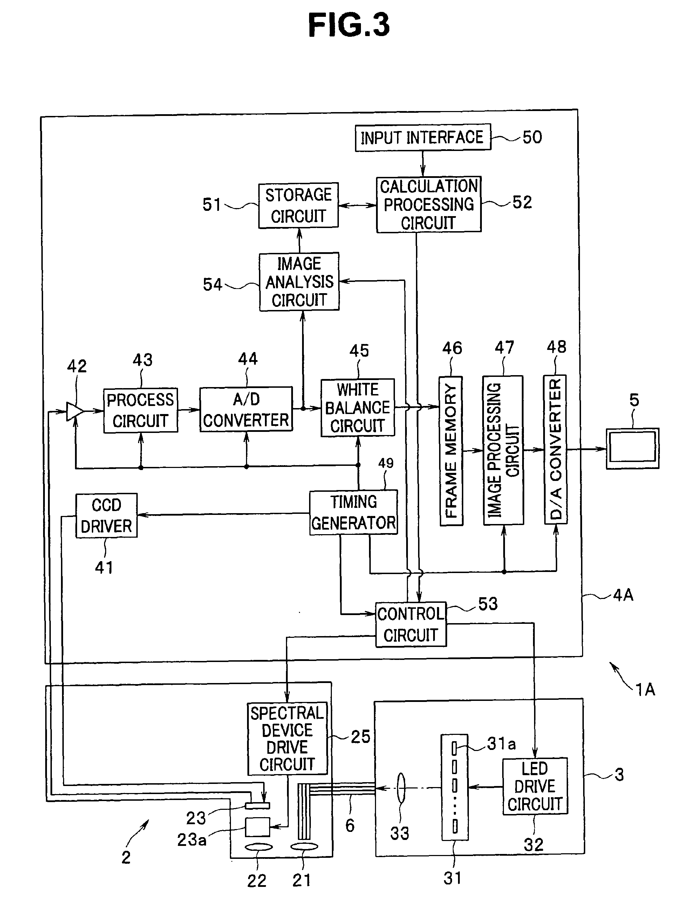

Fig. 3 to Fig. 11 are related to a second embodiment of the present invention.

In the following descriptions, the same components will be assigned the same reference numerals as those in the first embodiment and detailed descriptions thereof will be omitted. Furthermore, the configuration of the endoscope system in the present embodiment has a configuration similar to that of the first embodiment. Therefore, suppose the present embodiment will mainly describe differences from the first embodiment.

As shown in Fig. 3, an endoscope system 1A is configured by including an endoscope 2, a light source apparatus 3, a processor 4A and a monitor 5.

The processor 4A is configured as the processor 4 according to the first embodiment further provided with an image analysis circuit 54.

The image analysis circuit 54 analyzes an image signal outputted from an A/D converter 44 and then stores the analysis result in a storage circuit 51. Furthermore, the image analysis circuit 54 receives information on the control contents performed by a control circuit 53 as required.

Next, the operation of the endoscope system 1A will be described. Hereinafter, a case will be described where an observation is made using two types of fluorescence agents.

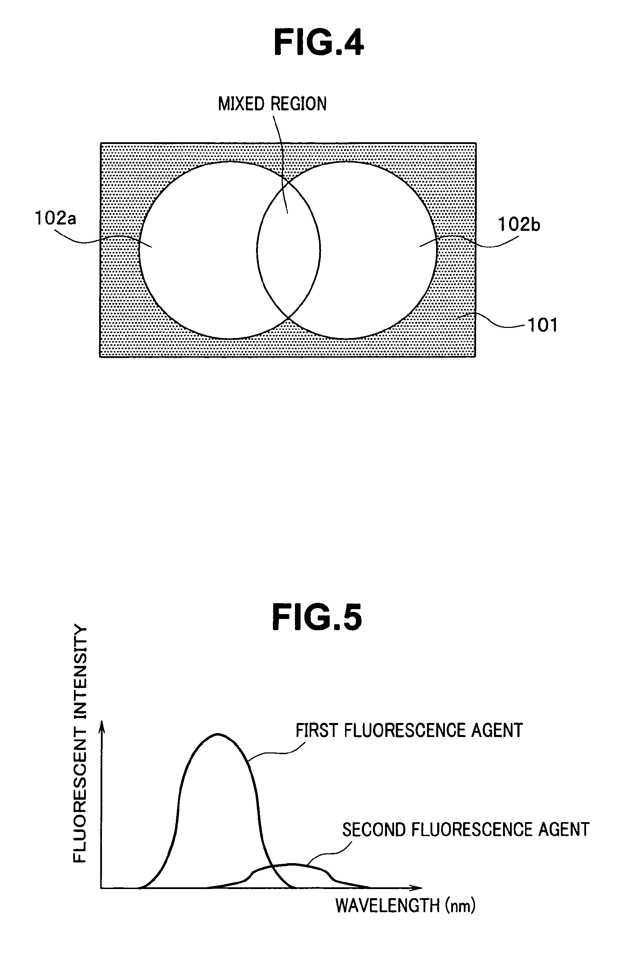

As shown in Fig. 4, the user uniformly applies a first fluorescence agent 102a and a second fluorescence agent 102b as two types of fluorescence agents used in combination in an actual observation over the surface of a non-fluorescence member 101. In this case, the user provides at least one mixed region where the first fluorescence agent 102a and the second fluorescence agent 102b are mixed together.

After that, the user places the non-fluorescence member 101 to which the first fluorescence agent 102a and the second fluorescence agent 102b have been applied, and an illumination optical system 21 and an objective optical system 22 so as to face each other.

The non-fluorescence member 101 to which the first fluorescence agent 102a and the second fluorescence agent 102b have been applied may also be placed on the bottom surface of a cap into which the distal end portion of the endoscope 2 can be inserted or may be formed into a flat plate shape.

The user then gives an instruction by operating an input interface 50 for optimizing the wavelength band of excitation light and the detection wavelength band of fluorescent light for each fluorescence agent.

A calculation processing circuit 52 reports that the aforementioned instruction has been given to the control circuit 53.

Upon detecting via the calculation processing circuit 52 that the aforementioned instruction has been given, the control circuit 53 performs control over an LED drive circuit 32 so as to sequentially turn ON LEDs 31a of an LED light source section 31 and also performs control over a spectral device drive circuit 25 so as to cause a spectral device 23a to intercept the wavelength band of excitation light emitted from the LEDs 31a which are ON. The control circuit 53 then outputs information on contents of the aforementioned control (timing at which each control is performed or the like) to the image analysis circuit 54 as required.

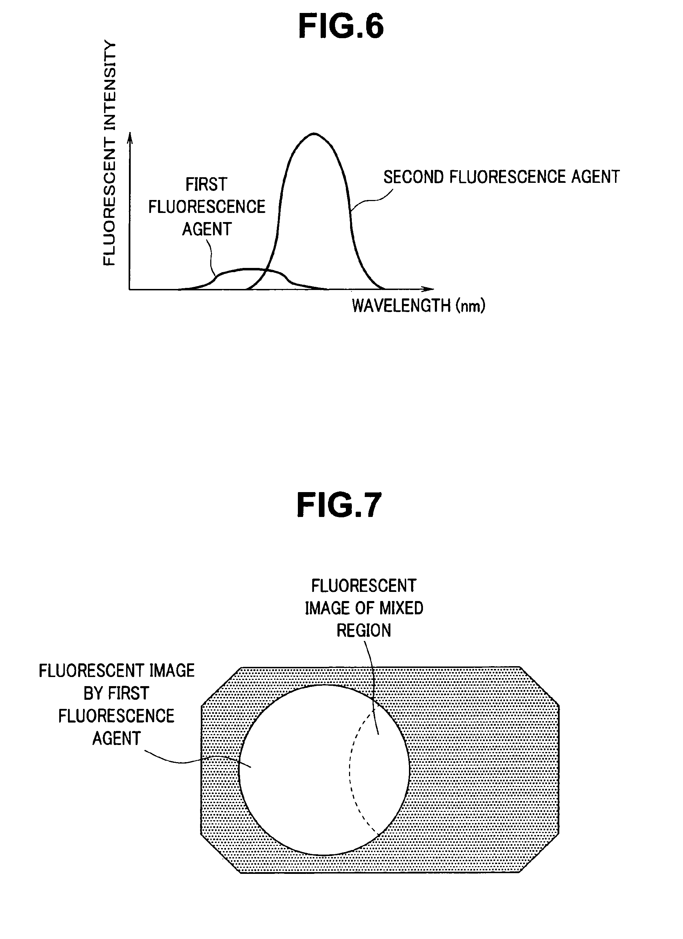

When the LEDs 31a of the LED light source section 31 are sequentially turned ON, the wavelength bands of the excitation light beams emitted to the first fluorescence agent 102a and the second fluorescence agent 102b are sequentially shifted. This causes the relationship between the intensity of the fluorescent light emitted from the first fluorescence agent 102a and the intensity of the fluorescent light emitted from the second fluorescence agent 102b to gradually change and, for example, in a case shown in Fig. 5, the intensity of the fluorescent light emitted from the first fluorescence agent 102a reaches a maximum value or in a case shown in Fig. 6, the intensity of the fluorescent light emitted from the second fluorescence agent 102b reaches a maximum value.

On the other hand, a CCD 23 acquires fluorescence images of the first fluorescence agent 102a and the second fluorescence agent 102b every time the wavelength band of excitation light shifts and sequentially outputs the acquired fluorescence images as image pickup signals.

The image pickup signal outputted from the CCD 23 is amplified by an amplifier 42, subjected to signal processing by a process circuit 43, converted to an image signal by the A/D converter 44 and then outputted to the image analysis circuit 54.

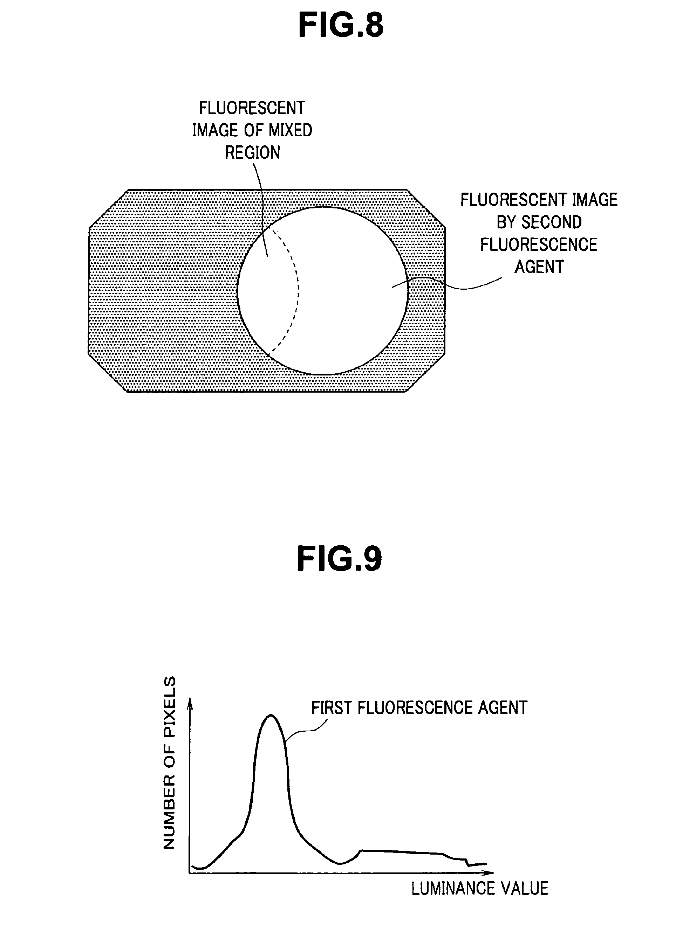

The image analysis circuit 54 sequentially analyzes each image signal inputted through the A/D converter 44 and thereby detects that fluorescence images, for example, as shown in Fig. 7 and Fig. 8 are acquired.

Here, the fluorescence image shown in Fig. 7 is obtained in the case shown in Fig. 5 where the intensity of the fluorescent light emitted from the first fluorescence agent 102a reaches a maximum value. Thus, the image analysis circuit 54 obtains an analysis result indicating that a wavelength band $\lambda_{c1}$ when the fluorescence image shown in Fig. 7 is obtained is optimum as the wavelength band of excitation light for exciting the first fluorescence agent 102a based on the information on the control contents of the control circuit 53.

On the other hand, the fluorescence image shown in Fig. 8 is obtained in the case shown in Fig. 6 where the intensity of the fluorescent light emitted from the second fluorescence agent 102b reaches a maximum value. Thus, the image analysis circuit 54 obtains an analysis result indicating that a wavelength band $\lambda_{c2}$ when the fluorescence image shown in Fig. 8 is obtained is optimum as the wavelength band of excitation light for exciting the second fluorescence agent 102b based on the information on the control contents of the control circuit 53.

The image analysis circuit 54 causes the storage circuit 51 to store the wavelength band $\lambda_{c1}$ of the excitation light for exciting the first fluorescence agent 102a and the wavelength band $\lambda_{c2}$ of the excitation light for exciting the second fluorescence agent 102b.

On the other hand, the calculation processing circuit 52 reports that the analysis results of the wavelength bands $\lambda_{c1}$ and $\lambda_{c2}$ by the image analysis circuit 54 have been stored in the storage circuit 51 to the control circuit 53.

Upon detecting via the calculation processing circuit 52 that the analysis results of the wavelength bands $\lambda_{c1}$ and $\lambda_{c2}$ by the image analysis circuit 54 have been stored in the storage circuit 51, the control circuit 53 performs control over the LED drive circuit 32 so as to emit white color light including the wavelength bands $\lambda_{c1}$ and $\lambda_{c2}$ from the LED light source section 31 and also performs control over the spectral device drive circuit 25 so as to gradually shift the transmission wavelength band of the spectral device 23a. The control circuit 53 then outputs information on contents of the aforementioned control (timing at which each control is performed or the like) to the image analysis circuit 54 as required.

When the white color light beams including the wavelength bands $\lambda_{c1}$ and $\lambda_{c2}$ are emitted from the LED light source section 31, fluorescent light beams are simultaneously emitted from the first fluorescence agent 102a and the second fluorescence agent 102b. When the transmission wavelength band of the spectral device 23a is gradually shifted in such a state, images of the object having the transmission wavelength band are sequentially formed on the image pickup surface of the CCD 23.

The CCD 23 acquires fluorescence images of the first fluorescence agent 102a and fluorescence images of the second fluorescence agent 102b every time the transmission wavelength band of the spectral device 23a is shifted and sequentially outputs the acquired fluorescence images as image pickup signals.

The image pickup signal outputted from the CCD 23 is amplified by the amplifier 42, subjected to signal processing by the process circuit 43, converted to an image signal by the A/D converter 44 and then outputted to the image analysis circuit 54.

The image analysis circuit 54 sequentially analyzes respective image signals inputted through the A/D converter 44 and thereby acquires spectral images corresponding to the respective image signals.

After that, in each spectral image, the image analysis circuit 54 detects a luminance value $B_1$ of the fluorescence image of a first region to which only the first fluorescence agent 102a is applied, a luminance value $B_2$ of the fluorescence image of a second region to which only the second fluorescence agent 102b is applied and a luminance value $B_m$ of the fluorescence image of the mixed region in which the first fluorescence agent 102a and the second fluorescence agent 102b are mixed together, respectively.

Next, the image analysis circuit 54 calculates a luminance ratio $CN_1$ in each image signal inputted to the image analysis circuit 54 by a calculation using following equation (4).

$$CN_1 = (B_m\text{-}B_1)/(B_m\text{+}B_1) \ldots (4)$$

Based on the calculation result of the luminance ratio $CN_1$ and the information on the control contents of the control circuit 53, the image analysis circuit 54 obtains an analysis result that when a spectral image is obtained in which the luminance ratio $CN_1$ becomes equal to or below a predetermined value $Q_{th}$, the transmission wavelength band $\lambda_{d1}$ in this case is optimum as the detection wavelength band to detect the fluorescent light emitted from the first fluorescence agent 102a.

Next, image analysis circuit 54 calculates a luminance ratio $CN_2$ in each image signal inputted to the image analysis circuit 54 by a calculation using following equation (5).

$$CN_2 = (B_m\text{-}B_2)/(B_m\text{+}B_2) \ldots (5)$$

Based on the calculation result of the luminance ratio $CN_2$ and the information on the control contents of the control circuit 53, the image analysis circuit 54 obtains an analysis result that when a spectral image is obtained in which the luminance ratio $CN_2$ becomes equal to or below a predetermined value $R_{th}$, the transmission wavelength band $\lambda_{d2}$ in this case is optimum as the detection wavelength band to detect the fluorescent light emitted from the second fluorescence agent 102b.

The image analysis circuit 54 causes the storage circuit 51 to store the transmission wavelength band $\lambda_{d1}$ to detect the fluorescent light emitted from the first fluorescence agent 102a and the transmission wavelength band $\lambda_{d2}$ to detect the fluorescent light emitted from the second fluorescence agent 102b.

Upon receiving an instruction for using two types of fluorescence agents; the first fluorescence agent 102a and the second fluorescence agent 102b in combination through an operation of the input interface 50, the calculation processing circuit 52 reads the wavelength bands $\lambda_{c1}$ and $\lambda_{c2}$ of the excitation light and the transmission wavelength bands $\lambda_{d1}$ and $\lambda_{d2}$ from the storage circuit 51 and then associates these wavelength bands with each other in a one-to-one correspondence and outputs the association result to the control circuit 53.

Based on input timing of a timing signal from the timing generator 49 and the wavelength bands $\lambda_{c1}$ and $\lambda_{c2}$ of the excitation light outputted from the calculation processing circuit 52, the control circuit 53 performs control over the LED drive circuit 32 so as to switch between the LEDs 31a to emit light in that order. Furthermore, based on input timing of a timing signal from the timing generator 49 and the transmission wavelength bands $\lambda_{d1}$ and $\lambda_{d2}$ outputted from the calculation processing circuit 52, the control circuit 53 performs control over the spectral device drive circuit 25 so as to switch the transmission wavelength bands of the spectral device 23a in that order.

Furthermore, the control circuit 53 synchronizes the timing of control over the LED drive circuit 32 with the timing of control over the spectral device drive circuit 25 based on input timing of a timing signal from the timing generator 49. According to such control of the control circuit 53, the operation of the LED light source section 31 of the light source apparatus 3 of emitting excitation light having a wavelength band $\lambda_{c1}$ is interlocked with the operation of the spectral device 23a of the endoscope 2 of allowing light (fluorescence image) having a transmission wavelength band $\lambda_{d1}$ to pass for the first fluorescence agent 102a. Furthermore, according to the aforementioned control of the control circuit 53, the operation of the LED light source section 31 of the light source apparatus 3 of emitting excitation light having a wavelength band $\lambda_{c2}$ is interlocked with the operation of the spectral device 23a of the endoscope 2 of allowing light (fluorescence image) having a transmission wavelength band $\lambda_{d2}$ to pass for the second fluorescence agent 102b.

On the other hand, the user inserts the insertion portion of the endoscope 2 into the body cavity of the examinee and places the distal end portion of the endoscope 2 so that excitation light is emitted to a position where a desired object such as cancer exists.

Furthermore, the user scatters or injects the first fluorescence agent 102a and the second fluorescence agent 102b whose information is inputted through the operation of the input interface 50 into the desired object using a treatment instrument or the like (not shown) having a shape and size that allows it to be inserted into the endoscope 2.

Every time excitation light beams of the wavelength bands $\lambda_{c1}$ and $\lambda_{c2}$ are sequentially emitted to the desired object, two types of fluorescent light corresponding to the respective excitation light beams are sequentially emitted from the desired object.

The fluorescent light beams emitted from the desired object pass through the objective optical system 22 and the spectral device 23a and sequentially form images on the image pickup surface of the CCD 23 as fluorescence images of the object having transmission wavelength bands $\lambda_{d1}$ and $\lambda_{d2}$.

The CCD 23 then sequentially generates image pickup signals corresponding to the fluorescence images of the object having the transmission wavelength bands $\lambda_{d1}$ and $\lambda_{d2}$ and outputs the image pickup signals to the processor 4A.

Each image pickup signal outputted from the CCD 23 is amplified by the amplifier 42, subjected to signal processing by the process circuit 43 and converted to a digital image signal by the A/D converter 44. Each image signal outputted from the A/D converter 44 is subjected to white balance processing by the white balance circuit 45 and then inputted to the frame memory 46.

The frame memory 46 sequentially stores image signals corresponding to each of the fluorescence images of the object having the transmission wavelength bands $\lambda_{d1}$ and $\lambda_{d2}$ in units of one frame.

The image processing circuit 47 simultaneously reads latest image signals corresponding to one frame for the fluorescence images of the object having the transmission wavelength bands $\lambda_{d1}$ and $\lambda_{d2}$ respectively based on input timings of timing signals from the timing generator 49 from the frame memory 46. The image processing circuit 47 then applies image processing such as coloring with different colors to each image signal read from the frame memory 46 and outputs each image signal to the D/A converter 48.

The D/A converter 48 converts each image signal outputted from the image processing circuit 47 to an analog video signal based on input timing of a timing signal from the timing generator 49 and outputs the video signal. Thus, the monitor 5 displays an image in which it is possible to visually recognize a distribution of fluorescent light beams emitted from the first fluorescence agent 102a and the second fluorescence agent 102b individually.

When a spectral image is obtained in which the luminance ratio $CN_1$ in equation (4) above becomes equal to or below a predetermined value $Q_{th}$, the present embodiment selects one wavelength band in which a luminance value $B_1$ of the fluorescence image takes a maximum value out of the respective transmission wavelength bands in this case as a transmission wavelength band $\lambda_{d1}$, and can thereby detect fluorescent light emitted from the first fluorescence agent 102a and make contrast compatible with brightness in the fluorescence image obtained.

Furthermore, when a spectral image is obtained in which the luminance ratio $CN_2$ in equation (5) above becomes equal to or below a predetermined value $R_{th}$, the present embodiment selects one wavelength band in which a luminance value $B_2$ of the fluorescence image takes a maximum value out of the respective transmission wavelength bands in this case as a transmission wavelength band $\lambda_{d2}$, and can thereby detect fluorescent light emitted from the second fluorescence agent 102b and make contrast compatible with brightness in the fluorescence image obtained.

On the other hand, when determining a transmission wavelength band of the spectral device 23a based on a spectral image corresponding to each image signal inputted through the A/D converter 44, the image analysis circuit 54 of the present embodiment may also perform processing which will be described below.

The image analysis circuit 54 acquires a luminance distribution expressed by a correlation between the luminance value and the number of pixels for each spectral image.

Here, a case of the aforementioned luminance distribution where the luminance value of one spectral image is set on the horizontal axis and the number of pixels corresponding to the luminance value is set on the vertical axis will be described as an example below.

The image analysis circuit 54 assumes as one peak, a portion in a luminance distribution of one spectral image provided with a point at which a differential value obtained by differentiating the luminance distribution with respect to the luminance value (value set on the horizontal axis) changes from positive to negative and where the number of pixels included within a predetermined range of luminance values becomes equal to or above a reference value.

The image analysis circuit 54 identifies peaks in the luminance distribution obtained for each spectral image using the aforementioned method and thereby obtains a luminance distribution provided with only one peak corresponding to fluorescent light emitted from the first fluorescence agent 102a as shown, for example, in Fig. 9.

The luminance distribution in Fig. 9 is obtained when a greater amount of fluorescent light emitted from the first fluorescence agent 102a is detected, as well as when substantially no fluorescent light emitted from the second fluorescence agent 102b or mixed light of these two fluorescent light beams is detected. Thus, based on the result of acquisition of the luminance distribution and information on the control contents of the control circuit 53, the image analysis circuit 54 obtains an analysis result that the transmission wavelength band $\lambda_{e1}$ when a luminance distribution provided with only one peak corresponding to fluorescent light emitted from the first fluorescence agent 102a is obtained is optimum as the detection wavelength band to detect fluorescent light emitted from the first fluorescence agent 102a.

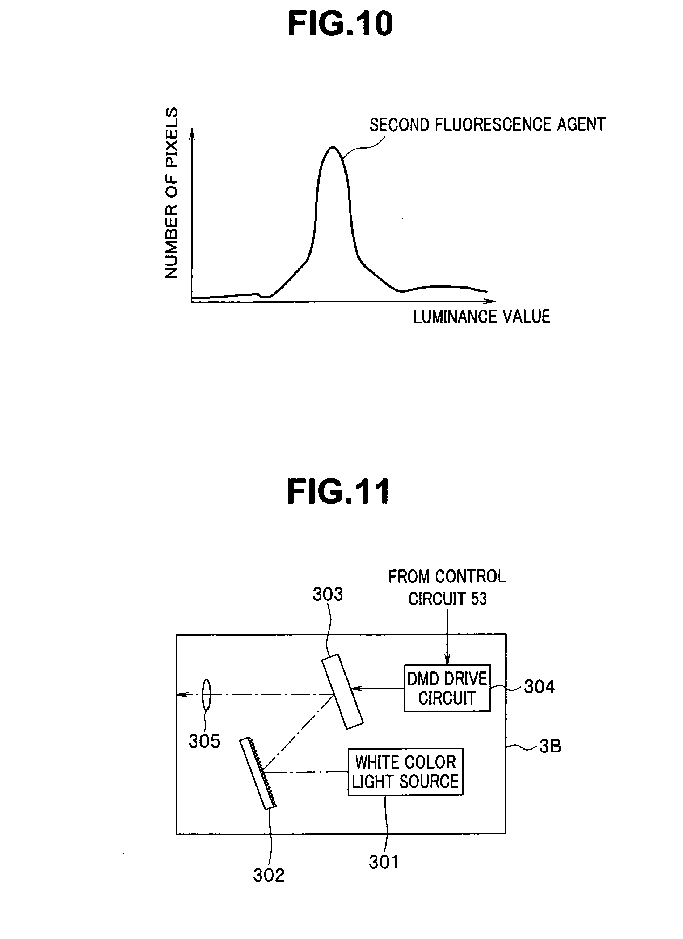

Furthermore, the image analysis circuit 54 identifies peaks in the luminance distribution obtained for each spectral image using the aforementioned method and thereby obtains a luminance distribution provided with only one peak corresponding to fluorescent light emitted from the second fluorescence agent 102b as shown, for example, in Fig. 10.

The luminance distribution in Fig. 10 is obtained when a greater amount of fluorescent light emitted from the second fluorescence agent 102b is detected, as well as when substantially no fluorescent light emitted from the first fluorescence agent 102a or mixed light of these two fluorescent lights is detected. Thus, based on the result of acquisition of the luminance distribution and information on the control contents of the control circuit 53, the image analysis circuit 54 obtains an analysis result that the transmission wavelength band $\lambda_{e2}$ when a luminance distribution provided with only one peak corresponding to fluorescent light emitted from the second fluorescence agent 102b is obtained is optimum as the detection wavelength band to detect fluorescent light emitted from the second fluorescence agent 102b.

A series of the aforementioned operations and processes can be applied in substantially the same way in the present embodiment by substituting the transmission wavelength band $\lambda_{d1}$ with $\lambda_{e1}$ and substituting the transmission wavelength band $\lambda_{d2}$ with $\lambda_{e2}$.

As described above, when making fluorescence observations using a plurality of fluorescence agents, the endoscope system 1A optimizes wavelength bands of excitation light and detection wavelength band of fluorescent light for each fluorescence agent, and can thereby acquire an image in which degradation of contrast caused by a cross-talk phenomenon is drastically suppressed. That is, the endoscope system 1A can reduce a cross-talk phenomenon generated when observing each corresponding fluorescent light emitted from a plurality of fluorescence agents in combination of various fluorescence probes.

Furthermore, the endoscope system 1A acquires an image of an object to which a combination of fluorescence agents used for observation is actually applied and then optimizes the wavelength bands of excitation light and detection wavelength bands of fluorescent light according to the analysis result of the object image. Thus, when using a fluorescence agent whose detailed fluorescence characteristics are unknown in combination, the endoscope system 1A can optimize center wavelengths of excitation light and detection wavelengths of fluorescent light and acquire an image in which degradation of contrast caused by a cross-talk phenomenon is drastically suppressed.

The endoscope system 1A of the present embodiment may also be configured using a light source apparatus 3B shown in Fig. 11 instead of the light source apparatus 3 shown in Fig. 3.

The light source apparatus 3B includes a white color light source 301, a diffraction grating 302 that causes white color light emitted from the white color light source 301 to diffract, a digital micro mirror device (hereinafter abbreviated as "DMD") 303 that reflects the light dispersed after passing through the diffraction grating 302, a DMD drive circuit 304 that drives the DMD 303 based on the control of the control circuit 53 and a collimate lens 305 that transforms the light reflected by the DMD 303 into parallel light and supplies the parallel light to the light guide 6.

The DMD 303 is formed by including a plurality of micro mirrors arranged in a grid-like form and has a configuration that allows orientation to be changed for each micro mirror according to the drive control of the DMD drive circuit 304.

According to the light source apparatus 3B having the above described configuration, the DMD drive circuit 304 performs such drive control as to individually assign a wavelength of light dispersed by the diffraction grating 302 to each micro mirror of the DMD 303 and reflect the light, and can thereby supply the parallel light dispersed for each wavelength included in white color light emitted from the white color light source 301 to the light guide 6.

In the fluorescence image obtained when the aforementioned parallel light is emitted to the first fluorescence agent 102a, the luminance value of the portion excited by the excitation light having the aforementioned wavelength band $\lambda_{c1}$ becomes highest. Furthermore, in the fluorescence image obtained when the aforementioned parallel light is emitted to the second fluorescence agent 102b, the luminance value of the portion excited by the excitation light of the aforementioned wavelength band $\lambda_{c2}$ becomes highest.

That is, when the light source apparatus 3B having the above described configuration is used, it is possible to drastically reduce the number of times fluorescence images are acquired when the aforementioned wavelength bands $\lambda_{c1}$ and $\lambda_{c2}$ are determined.

It goes without saying that the present invention is not limited to the aforementioned embodiments but can be modified and applied in various ways without departing from the spirit and scope of the present invention.

The present application is filed claiming the priority of Japanese Patent Application No. 2009-072167 filed in Japan on March 24, 2009, and the above described disclosure is incorporated by reference in the present description, claims and drawings.

**Claims**

1. A fluorescence observation apparatus comprising:

   a light source section that can emit a plurality of excitation light beams of different wavelength bands and select excitation light beams to be emitted to an object where there are a first fluorescent substance and a second fluorescent substance from among the plurality of excitation light beams;
   a variable filter section that selectively allows at least part of the wavelength band of returning light from the object to pass;
   a photo-detecting section that detects the light that has passed through the variable filter section;
   a calculation processing section that carries out calculations to determine a wavelength band of the excitation light emitted from the light source section and a transmission wavelength band in the variable filter section based on intensity characteristics of the plurality of excitation light beams, fluorescence characteristics of the first fluorescent substance and fluorescence characteristics of the second fluorescent substance; and
   a control section that performs control of causing the light source section to interlock with the variable filter section based on the calculation result of the calculation processing section.

2. The fluorescence observation apparatus according to claim 1, further comprising a storage section that stores a plurality of fluorescence characteristics including fluorescence characteristics of the first fluorescent substance and fluorescence characteristics of the second fluorescent substance beforehand,
   wherein the calculation processing section reads the fluorescence characteristics of the first fluorescent substance and the fluorescence characteristics of the second fluorescent substance from the storage section based on information inputted by an input section before performing the calculation.

3. The fluorescence observation apparatus according to claim 1, wherein the control section performs, based on the calculation result of the calculation processing section, control of synchronizing, timing of emitting first excitation light for exciting the first fluorescent substance from the light source section with timing of causing the wavelength band of the first fluorescent light emitted from the first fluorescent substance to pass through the variable filter section, and synchronizing timing of emitting second excitation light for exciting the second fluorescent substance from the light source section with timing of causing the wavelength band of second fluorescent light emitted from the second fluorescent substance to pass through the variable filter section.

4. A fluorescence observation apparatus comprising:

a light source section that can emit a plurality of excitation light beams of different wavelength bands and select excitation light beams to be emitted to an object where there are a first fluorescent substance and a second fluorescent substance from among the plurality of excitation light beams;

a variable filter section that selectively allows at least part of the wavelength band of returning light from the object to pass;

a photo-detecting section that detects the light that has passed through the variable filter section;

an analysis section that acquires a fluorescence image corresponding to the fluorescent light emitted from the first fluorescent substance, a fluorescence image corresponding to the fluorescent light emitted from the second fluorescent substance and a fluorescence image corresponding to the fluorescent light emitted from a mixed region of the first fluorescent substance and the second fluorescent substance based on the detection result in the photo-detecting section and determines a wavelength band of the excitation light emitted from the light source section and a transmission wavelength band in the variable filter section based on luminance of each of the fluorescence images; and

a control section that performs control of causing the light source section to interlock with the variable filter section based on the analysis result of the analysis section.

5. The fluorescence observation apparatus according to claim 4, wherein the control section performs, based on the calculation result of the calculation processing section, control of synchronizing, timing of emitting first excitation light for exciting the first fluorescent substance from the light source section with timing of causing the wavelength band of the first fluorescent light emitted from the first fluorescent substance to pass through the variable filter section, and synchronizing timing of emitting second excitation light for exciting the second fluorescent substance from the light source section with timing of causing the wavelength band of second fluorescent light emitted from the second fluorescent substance to pass through the variable filter section.

# FIG.1

# FIG.2

FROM CONTROL CIRCUIT 53

SPECTRAL
DEVICE
DRIVE
CIRCUIT ~ 36

~ 3A

LAMP ~ 34

37

35

# FIG.3

INPUT INTERFACE — 50

51 — STORAGE CIRCUIT

CALCULATION PROCESSING CIRCUIT — 52

54 — IMAGE ANALYSIS CIRCUIT

42

43 — PROCESS CIRCUIT

44 — A/D CONVERTER

45 — WHITE BALANCE CIRCUIT

46 — FRAME MEMORY

47 — IMAGE PROCESSING CIRCUIT

48 — D/A CONVERTER

5

CCD DRIVER
41

49 — TIMING GENERATOR

CONTROL CIRCUIT — 53

4A

1A

SPECTRAL DEVICE DRIVE CIRCUIT — 25

31a

LED DRIVE CIRCUIT — 3

23
23a

2

22    21

6

33

31

32

# FIG.4

MIXED REGION

102a

102b

101

# FIG.5

FLUORESCENT INTENSITY

FIRST FLUORESCENCE AGENT

SECOND FLUORESCENCE AGENT

WAVELENGTH (nm)

# FIG.6

# FIG.7

# FIG.8

FLUORESCENT
IMAGE OF MIXED
REGION

FLUORESCENT IMAGE
BY SECOND
FLUORESCENCE
AGENT

# FIG.9

FIRST FLUORESCENCE AGENT

NUMBER OF PIXELS

LUMINANCE VALUE

EP 2 412 289 A1

# FIG.10

# FIG.11

**INTERNATIONAL SEARCH REPORT**

| International application No. |
|---|
| PCT/JP2009/067666 |

A. CLASSIFICATION OF SUBJECT MATTER
*A61B1/00*(2006.01)i

According to International Patent Classification (IPC) or to both national classification and IPC

B. FIELDS SEARCHED

Minimum documentation searched (classification system followed by classification symbols)
A61B1/00, G01N21/64

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | US 5413108 A (Robert R. Alfano), 09 May 1995 (09.05.1995), column 4, line 15 to column 5, line 31; fig. 3 (Family: none) | 1-5 |

☐ Further documents are listed in the continuation of Box C.  ☐ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| 03 December, 2009 (03.12.09) | 15 December, 2009 (15.12.09) |

| Name and mailing address of the ISA/ <br> Japanese Patent Office | Authorized officer |
|---|---|
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008043396 A
- JP 2009072167 A